# EUROPEAN PATENT APPLICATION

(11) **EP 4 216 144 A1**
(43) Date of publication of application: **26.07.2023**
(21) Application number: 21868787.9
(22) Date of filing: 20.09.2021
(51) Int. Cl.: G06Q 50/00, H04W 4/02, G08B 21/00, H01M 8/1286

(54) **DEVICE FOR PASSIVE DETECTION OF EVENTS**

(30) Priority: 18.09.2020 ES 202030949
(71) Applicant: Consejo Superior de Investigaciones Científicas (CSIC), 28006 Madrid (ES); Institució Catalana De Recerca I Estudis Avançats (ICREA), 08010 Barcelona (ES); Fuelium, SL, 08193 Bellaterra (Barcelona) (ES); Institut de Fisica d'Altes Energies (IFAE), 08193 Bellaterra, Barcelona (ES)
(72) Inventor: CASTELLARNAU, Marc, 08193 Bellaterra (Barcelona) (ES); MACIAS, Jose Gabriel, 08193 Bellaterra (Barcelona) (ES); SABATE, Neus, 08010 Barcelona (ES); ESQUIVEL, Juan Pablo, 08193 Bellaterra (Barcelona) (ES)
(74) Representative: Pons
(86) International application number: PCT/ES2021/070679
(87) International publication number: WO 2022/058639

(57) **Abstract**

That consumes zero power until an alarming event takes place, as it takes advantage of the phase transition of a PCM material (2) (Phase Change Material) to monitor relevant physical/chemical events. If the PCM material (2) is ion-conducting, the device comprises a liquid-activated battery (1), an ion-conducting PCM material (2) (Phase Change Material), positioned in contact with the liquid-activated battery (1), and an electronics module (4), connected to the liquid-activated battery (1), that powers up with the battery. If the PCM material (2) is a non ion-conducting PCM material (2), the device additionally comprises a ion-conducting liquid, and being the non ion-conducting PCM material (2) placed as a barrier between the ion-conducting liquid and the liquid-activated battery (1).

## Description

### OBJECT OF THE INVENTION

The object of the invention is a passive event detection device, that belongs to the wireless-sensor-node area, in which a physical/chemical parameter is monitored continuously and communicated to a receiver in a remote manner. The device avoids current power-consuming for continuous monitoring of a physical magnitude, such as temperature, and implements a self-triggered strategy that consumes absolutely no power until relevant information from the ambient is needed to be either stored and/or reported.

### BACKGROUND OF THE INVENTION

It is undeniable that the fulfilment of the Internet-of Things scenario, which is enabled by multiple energy autonomous systems that are comprehensively capable of sensing, diagnosing, deciding and actuating in a communicative and collaborative way, is leading society towards a digitalized new era.

Although loT is still in the early stages of growth, current estimations point to more than nine billion connected devices around the world. This number is expected to increase exponentially, with estimates ranging from 25 billion to 50 billion devices in 2025.

Energy autonomy of sensing nodes has been largely identified as a key enabling feature of digital scenario and till now, significant financial and technological efforts to obtain sustainable power sources able to harvest energy from the environment (light, heat and movement) have been invested during the last decade.

Yet, state-of-the-art autonomous sensing devices make use of batteries, as they are the sole candidates to provide sufficient power output in a reliable manner whereas the energy harvester elements (solar cells, thermoelectric generators, piezoelectric generators, etc...) are used to expand the limited operation time of such batteries. However, it is unclear whether the energy needs of billions of power-hungry devices are to be satisfied with current battery technologies, as demands forecasts in growing sectors like transportation and computing, which seem to compromise the availability of lithium and other key battery materials.

In particular, Internet of Things (IoT) can be defined as a network of nodes able to monitor physical and chemical magnitudes, provide relevant information concerning safety of individuals and goods and if required, perform an actuation. This involves continuous monitoring to assess that a safety-relevant magnitude has not reached an alarming threshold value, and therefore a continuously consuming energy to monitor their surroundings.

Despite all the efforts directed to develop low-power sensing and communicating electronic devices, the upcoming implementation of the Internet-of-Things (IoT) scenario will entail a dramatic increase in the energy needs that will not be met with the solely use of portable batteries. Moreover, many of the loT systems are intended to surveil single unwanted events such as water flooding, fire, vacuum breaks, toxic gas presence, etc., that take place seldomly along the operational life of the device. This means that most of these systems make use of electrical power to monitor continuously the absence of relevant events.

Stable and reliable power sources are key factors to ensure a monitoring scenario of several months to years. Significant efforts of continuously harvesting energy from the environment have been done in the last decade. Solar, flow systems (wind and hydro), heat and mechanical harvesting have evolved to provide sufficient power to enable wireless communication under controlled ambient conditions.

Nevertheless, their dependence on environmental conditions constrains their application in a very limited range of settings. This makes primary batteries the most preferred power for wireless sensor networks and today, the optimization of low-power consumption modules allows expanding power autonomy of wireless nodes up to several years.

However, the current predominance of primary batteries in the IoT scenario and the expected strong market penetration of sensing modules allow foreseeing a huge production, usage and disposability of battery components that depicts a rather non-ecofriendly panorama. Moreover, the need of a power source supposes a great limitation in terms of autonomy for those sensors placed at a remote location.

The technologies used to perform the detection are diverse, and also the communication strategies utilized to send the recorded signal (depending on the distance to receiver). Moreover, all of them need energy to function in a continuous way. Some of these devices are connected to the electricity network whereas others need a stand-alone battery due to their location. The need of a power source is their common feature.

As a particular example, fire detection and monitoring in forests is realized by three main methods: using optical cameras; sensing with wireless sensor networks the humidity, the composition, and the temperature of the air; and analyzing infrared imaging data from geostationary satellites every 30 or 15 minutes with limited spatial resolution of 3 km. Fire detection in buildings is realized by the detection of smoke and/or very high temperature detectors through active fire detection systems usually placed in the ceilings in every room.

Therefore, a very large amount of energy is wasted, as all of these devices work constantly in a standby state. Moreover, when there is the need for very precise measurements, these systems are constantly checking their surroundings. When the systems require less precise measurements, the systems use less energy by only checking once in a while, and therefore, loosing relevant measurements.

There is a need for a device which combines a zero energy waste, as well as permanent monitoring, in order to avoid constantly draining power from a battery and losing any relevant measure.

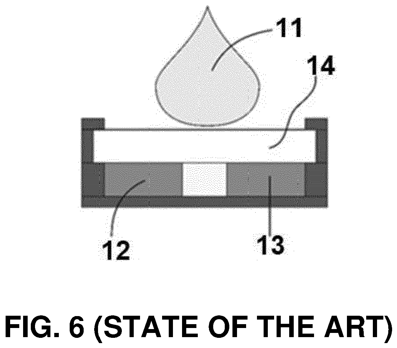

Apart from that, liquid-activated batteries are devices that consist of at least two electroactive electrodes, at least one of them oxidizing (anode 12) and at least one of them reducing (cathode 13) connected by a hydrophilic and/or porous material (or a receptacle/cavity) 14 able to hold a fluid 11, as depicted in Fig. 6. The battery starts to function upon the addition of the fluid 11, as this fluid acts as the battery electrolyte. The liquid used to activate the battery operation is generally a water-based fluid.

These batteries are primary batteries that cease to function when one of the electrodes is exhausted. Their limited operating time and simplicity in terms of structure and materials used makes them particularly suitable for short-term applications such as diagnostic or wearable devices.

### DESCRIPTION OF THE INVENTION

The passive event detection device, object of the present invention, comprises a phase-change material (PCM) that responds to physical/chemical changes in the environment allowing the development of wireless sensing nodes with zero-power consumption during sensing. The energy of a liquid-activated battery is only used when an alarming change in the environment happens.

The device uses the solid to liquid phase changes of the PCM to convert a change in a physical/chemical parameter of the surrounding environment into electrical energy, by preferably using the originated liquid phase to activate a liquid-activated battery. The activation of the battery allows generating a pulse of electrical power that can be used to power up an electronics module.

Such electronics module can sense, actuate and/or inform about the status of the device and its surrounding environment. For example, the electronics module can generate and optionally send, an alarm signal to a remote receiver by radiofrequency, light, sound or any other way of data transmission. Alternatively, the electrical power generated in the battery can be used to light an informative display or geolocate the device.

In this way, the battery-PCM combination remains inactive until the PCM material harvests sufficient energy from the environment to perform the phase transition (i.e. become liquid). Contrarily to the current loT paradigm, this device would consume no power unless an alarming event takes place, thus using the electrochemical energy stored in the liquid-activated battery only when the system gathers relevant information to report.

The proposed device could be adapted and used in multiple applications depending on the parameter responsible for the phase-change transition of the PCM, which extends from physical parameters such as temperature, mechanical stress, hydrostatic pressure, electromagnetic field, electrical field, radiation, and radioactivity to chemical parameters such as gas composition, water absorption, pH, etc.

Such phase-change materials could generate passive alarm systems for detection of the associated physical/chemical parameters in buildings, parking areas, research facilities, factories, transportation, and forests among the most relevant. It could also be used, for example, for packaging monitoring (temperature, location, and humidity), cold-chain monitoring in the retail sector (pharma, chemical, food), fire detection in private or public buildings or open spaces (forest) or gas detection in professional environments (labs, clean rooms), monitoring on individuals of temperature, radiation levels, gas concentration for safety purposes (firemen or other workers under severe thermal, radiation, or chemical stress), among others.

The device has the advantages of being low cost, environmentally friendly and yet smart, because the approach simplifies the manufacturing and minimizes the amount and the diversity of the materials and the electrical components required to allow ubiquitous monitoring of large spaces for extended periods of time.

Particularly, the device comprises a liquid-activated battery and a PCM material positioned in contact with the liquid-activated battery. The device also comprises an electronics module, connected to the liquid-activated battery. In this way, when the PCM material changes from solid to liquid, the liquid-activated battery activates and the electronics module is powered up.

The electronics module can then store information in an internal or external memory, sense the environment (temperature, humidity, etc.), activate an actuator (valve, engine, etc.) or generate a signal, which can be noticed from the outside, or which can be sent to an external device. The information provided by the electronics module can be transmitted by any mean like radiofrequency waves, light, sound, vibration, or can be stored in the memory contained in the electronics module, for example.

The liquid-activated battery, the PCM material and the electronics module can be placed on a support substrate that can be rigid or flexible. The device can be fabricated in the form of a tag, that can be then attached to any surface.

Relating to the placement and the attributes of the PCM material regarding the liquid-activated battery, there are several embodiments. Firstly, the PCM material can be an ion-conducting material (electrolytic) in its liquid phase (non ion-conducting in its solid state) and in direct contact with the liquid-activated battery, either on the outside of the liquid-activated battery, or inside the liquid-activated battery and would act as the battery electrolyte upon the phase-change event of changing from solid to liquid.

Secondly, the PCM material can be a non ion-conducting PCM material, and in this case the device additionally comprises an ion-conducting liquid, intended to be the one that activates the liquid-activated battery. In this case, the non ion-conducting PCM material acts as a barrier between the ion-conducting liquid and the liquid-activated battery, allowing its activation when the non ion-conducting PCM material changes from solid to liquid, and the ion-conducting liquid contacts the liquid-activated battery.

In this case, there are also two possible embodiments. The ion-conducting liquid and the non ion-conducting PCM material are outside the liquid-activated battery, being, for example, the ion-conducting liquid stored in a container, and the non ion-conducting PCM material placed as a barrier separating the ion-conducting material from the liquid-activated battery.

In another embodiment, the non ion-conducting PCM material can be forming microcapsules which contain the ion-conducting liquid, and being the microcapsules placed in contact with the liquid-activated battery (outside) and/or inserted in the liquid-activated battery. In this way, when the non ion-conducting PCM material changes from solid to liquid, the ion-conducting liquid is released and activates the liquid-activated battery.

Alternatively, the non ion-conducting PCM material can be a substrate of a porous material, wherein the ion-conductive liquid is placed in the pores of the non ion-conducting PCM material. The non ion-conducting PCM material can be placed outside and in contact with the liquid-activated battery, or can be inserted in the liquid-activated battery.

The information to be sensed from the environment is coded in the properties of the PCM material, which can be adjusted and designed to trigger the material phase transition. The volume, geometry and the intrinsic physical and/or chemical properties of the PCM material are variables that allow tuning the ambient energy required for the PCM material to perform the phase transition. This can be used to adjust the PCM material characteristics to match a particular intensity and/or duration of the ambient parameter so the PCM material performs phase transition when the pre-set conditions are fulfilled.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1.- Shows a view of the deposit containing the ion-conducting liquid, separated from the liquid-activated battery by the non ion-conducting PCM material.
Figure 2.- Shows a general view of the device, in an embodiment of the invention.
Figure 3.- Shows the output voltage of temperature sensitive PCM-battery assemblies with different PCM materials that are triggered at different temperatures.
Figure 4.- Shows a diagram of the different electronic components of the electronics module integrated in an embodiment of the device.
Figure 5.- Shows the experimental validation of the device activation upon an alarming rise of ambient temperature.
Figure 6.- Shows a diagram of the liquid-activated battery from the state of the art.
Figure 7.- Shows a diagram of the different electronic components of the electronics module of the device shown if Figure 2.

### PREFERRED EMBODIMENT OF THE INVENTION

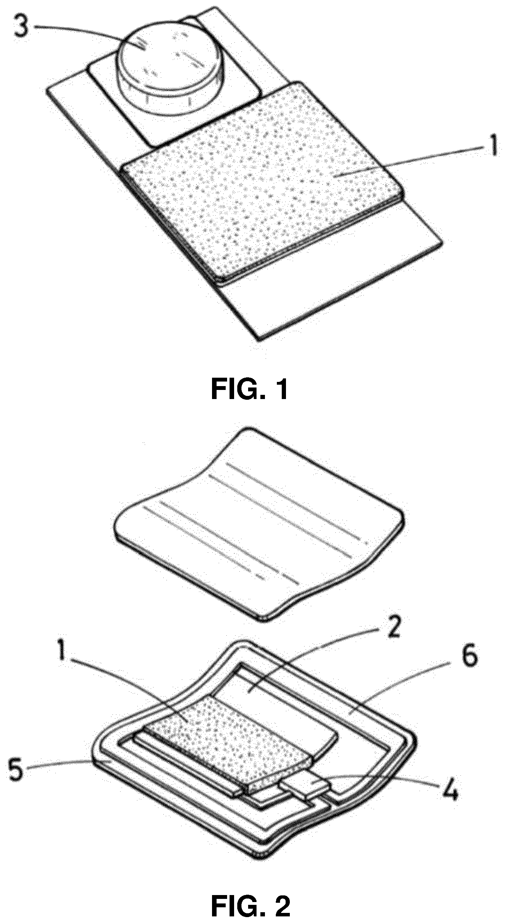

With help from figures 1 to 7, a preferred embodiment of the passive event detection device is described.

The passive event detection device, object of the present invention, comprises a liquid-activated battery (1) and a PCM material (2), positioned in contact with the liquid-activated battery (1). The liquid-activated battery (1) is a single use paper-battery. The device also comprises an electronics module (4), connected to the liquid-activated battery (1).

In this way, when the PCM material (2) changes from solid to liquid, the liquid-activated battery (1) activates and the electronics module (4) powers up. The electronics module (4) can comprise a memory, an emitter (6), and actuator or one or more sensors that activate when the electronics module (4) is powered up.

In this way, the electronics module (4) can store information in the memory, can perform an action with the actuator (open a valve, start and engine), can sense the environment (temperature, humidity, etc.) or can send a signal which can be visible from the outside, or which can be sent to an external device.

The signal emitted by the electronics module (4) can be a radiofrequency signal, a light, a sound, a vibration, or can be stored in a memory contained in the electronics module (4), for example.

The event that produced the change of the PCM material (2) from solid to liquid can be a change in temperature, light, pressure, gas concentration, radioactivity, etc. In this case, a change of temperature is going to be studied as the trigger of the PCM material (2).

The liquid-activated battery (1), the PCM material (2) and the electronics module (4) can be placed in a support substrate (5).

Relating the placement of the PCM material (2) with respect to the liquid-activated battery (1), there are several possible embodiments. Firstly, if the PCM material (2) is an ion-conducting PCM material (2) (being only ion-conducting in its liquid phase and non-ion conducting in its solid phase), it is placed in direct contact with the liquid-activated battery (1), either on the outside of the liquid-activated battery (1), or inside the liquid-activated battery (1), in the form of, for example, microcapsules inserted in the liquid-activated battery (1). In this case, the PCM material (2) is an ion conducting material, in order to be able to activate the liquid-activated battery (1) when it melts.

An example of this embodiment can be seen in figure 2, wherein the device comprises a flexible substrate (5), where the liquid-activated battery (1) and the ion-conducting PCM material (2) is in direct contact with the liquid-activated battery (1). The device also comprises an electronics module (4) connected to the liquid-activated battery (1) and an antenna (6), in order to send a signal to an external device when the battery (1) is activated.

Secondly, if the PCM material (2) is a non ion-conducting PCM material (2), the device comprises additionally an ion conducting liquid, intended to be the one that activates the liquid-activated battery (1). In this case, the PCM material (2) acts as a barrier between the ion-conducting liquid and the liquid-activated battery (1), allowing its activation when the non ion-conducting PCM material (2) melts and the ion-conducting liquid contacts the liquid-activated battery (1).

In this case, there are also two possible embodiments. The first one, shown in figure 1, is when the ion-conducting liquid and the non ion-conducting PCM material (2) are outside the liquid-activated battery (1), being, for example, the ion-conducting liquid stored in a container (3), a blister in this case, with the non ion-conducting PCM material (2) placed as a thin layer sealing the container (3), separating the ion-conducting liquid from the liquid-activated battery (1). When the ambient temperature crosses the phase transition temperature of the non ion-conducting PCM material (2), the blister releases the ion-conducting liquid and activates the liquid-activated battery (1), powering up the electronics module (4).

In the second embodiment, the non ion-conducting PCM material (2) is forming microcapsules, which contain the ion-conducting liquid, and being the microcapsules placed outside and in contact with the liquid-activated battery (1) and/or inserted in the liquid-activated battery (1). In this way, when the non ion-conducting PCM material (2) melts, the ion-conducting liquid activates the liquid-activated battery (1).

Alternatively, the non ion-conducting PCM material (2) can be a substrate of a porous material, wherein the ion-conductive liquid is placed in the pores of the non ion-conducting PCM material (2). The non ion-conducting PCM material (2) can be placed outside and in contact with the liquid-activated battery (1), or can be inserted in the liquid-activated battery (1).

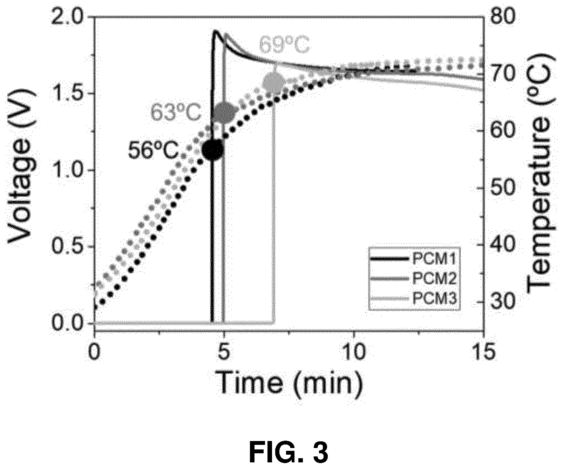

The PCM material (2) can be chosen to operate at different temperature ranges, with different phase transition temperatures, as shown in figure 3. The figure presents the operation of temperature sensitive-batteries customized to activate at three different temperatures. As it can be seen, the measured battery voltage in each device rises when the surrounding temperature reaches the PCM material (2) phase transition. The capability to define the trigger temperature based on the selection of the PCM material (2) makes this approach a versatile solution for different applications where temperature is a key factor.

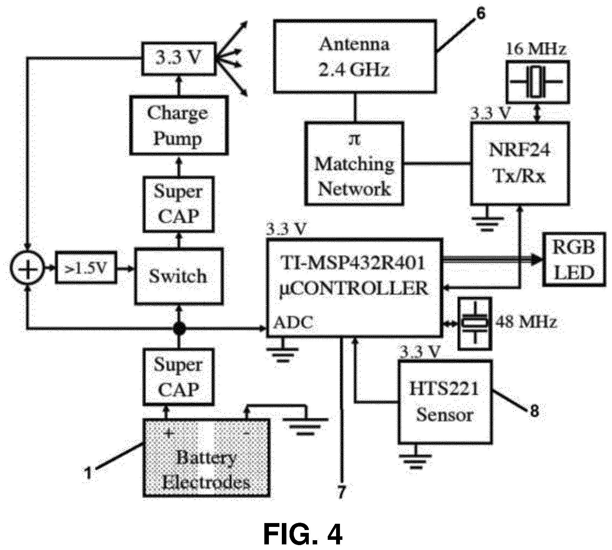

Particularly, in figure 3, solid lines represent battery voltage evolution; dotted-lines account for the measured temperature near the battery. Temperature values plotted with large dots indicate the ambient temperature at which the batteries with different PCM's are activated. The electronics module (4), shown in figure 4, performs three main functions: first, the battery's (1) voltage regulation and boost up to 3.3 V in order to bias commercial components comprised in the module; second, the monitoring of the temperature and the humidity of the device; and third, the connection to a master station (external device) using a dedicated point-to-point communications protocol.

The battery management section comprises, as shown in figure 4, a delayed and battery-level enabling a switch to accommodate for the battery's (1) activation dynamics and to provide power supply to all the electronics synchronously. A microcontroller (7) manages the operation of the complete electronics module (4). Every two seconds, it reads the measurements of a temperature and humidity sensor (8) located in the substrate (5) and sends the readout to an RF transceiver (10) that communicates to a master in the ISM band of 2.4 GHz.

The substrate (5) of the device can be of a tag format, as shown in figure 2, with a flexible surface. It can be made out of a compostable material. In this way, in case the device is used for monitoring fires in the woods, it will not pollute them. Moreover, if the device is used to monitor paper packages, they will still be recyclable, avoiding plastic material that could disturb the established recycling processes.

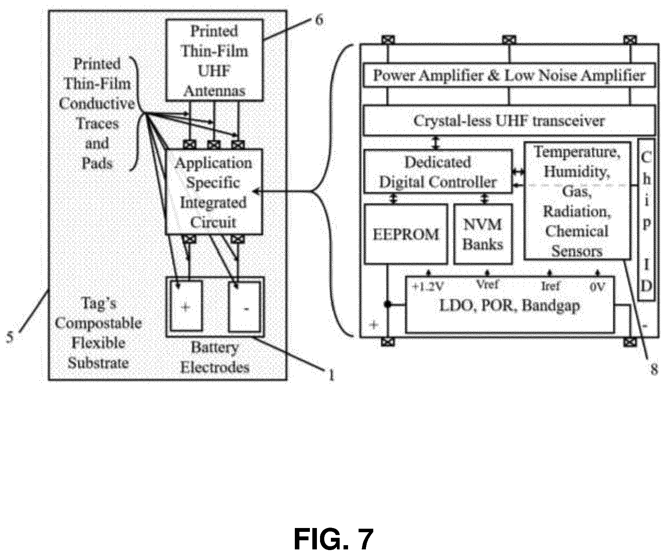

All the device components can be fabricated with printed and roll-to roll manufacturing including hybridization of the electronics module (4), which will be integrated into a single microelectronics chip to reduce cost, area, consumption, complexity and environmental impact upon disposal, as shown in figure 7.

In the embodiment were the device is compostable, the liquid-activated battery (1) is a paper-based battery, the substrate (5) is made out of a compostable material, and the electronics modules (4) comprises, as shown in figure 7, one or several thin-film antennas (6) (with carbon nanotubes or similar) printed on the biodegradable substrate (5), and an application specific integrated circuit (ASIC) without encapsulation connected to the antennas (6) and the liquid-activated battery (1).

The ASIC comprises a battery management unit, a dedicated digital controller with memory banks, physical and chemical sensing circuits, a chip ID, a crystal-less UHF transceiver and an unit with a power amplifier for the transmission and a low-noise-amplifier for the reception.

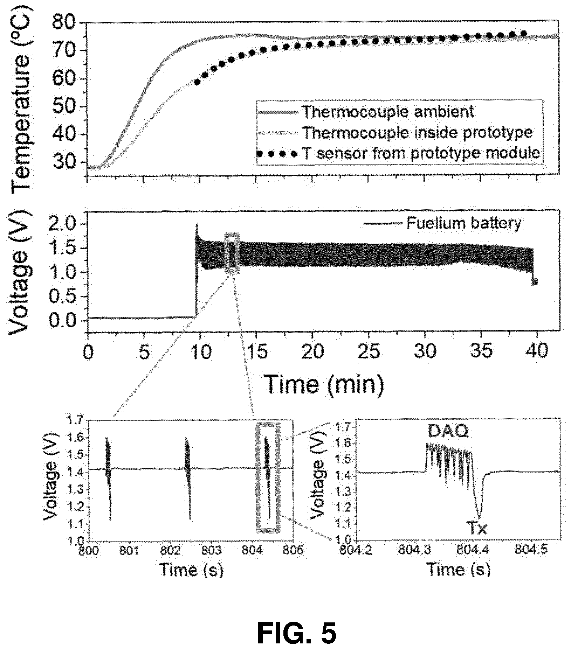

The validation of the device operation is shown in Figure 5. The passive event detection device was placed inside of an oven that was set to increase its temperature from 25 ºC to 70 ºC (oven temperature labelled as "thermocouple ambient"). For testing purposes, the battery (1) voltage was measured and an external thermocouple was inserted inside the device to record the temperature evolution (softer continuous line labelled as "Thermocouple inside prototype").

The device was customized to be triggered when temperature inside it exceeds 55 ºC. As it can be seen, the battery (1) voltage rises when the device reaches the set temperature. Once activated, the sensors (8) embedded in the electronics module (4) start recording the temperature, processing information and sending data wirelessly. A custom-made data receiver processes the information sent by the device.

The dotted line (labelled as "T sensor from prototype module") represents the temperature data received, which coincides with the temperature recorded with the external thermocouple. The figure insets show the fluctuations of battery (1) voltage due to the power consumption of the circuit during standby, data acquisition and data transmission.

The device can be applied in several fields, and some of them are explained bellow. In a scenario of fire detection, the device would only use energy from the battery (1) when the alarming event (sudden increase of temperature) turned the PCM material (2) from solid to liquid. The activation of the battery (1) would allow sending a signal from the electronics module (4) to a receiver placed at a long distance from the event. For example, in a mid-large fire in the forest, a population of devices distributed in the forest can provide accurate information about the dynamics of the fire during night when fire-brigade is not allowed to use aerial equipment to evaluate the direction and intensity of the fire. Such information is crucial to plan the fire extinction actions with better chances of success.

In the case of fire detection in buildings, the availability of the device placed in strategic places with higher inflammability probabilities would allow detecting fire at a very early stage. The device can be placed near the most probable sources of fire such as trash bins or electric panels, which will trigger much faster than smoke or fire detectors in the ceilings.

When applied to packages, the device can trigger if the package has been submitted to non-desired thermal conditions. For example, temperatures above 0° in case of frozen goods or temperatures above 30º in case of perishable chemical reactants.

## Claims

1. A non-active event detection device, that comprises:
- a support substrate (5),
- a liquid-activated battery (1), linked to the support substrate (5),
- an ion-conducting liquid,
- a non-ion conducting PCM material (2) (Phase Change Material), placed as a physical barrier between the ion-conducting liquid and the liquid-activated battery (1), that changes from solid to liquid when a physical or chemical environmental magnitude exceeds a threshold in intensity and/or duration, and
- an electronics module (4), connected to the liquid-activated battery (1), that is powered up when the liquid-activated battery (1) is activated.

2. The device according to claim 1, wherein the device additionally comprises a container (3) linked to the support substrate (5), for containing the ion-conducting liquid, being the non ion-conducting PCM material (2) placed as a barrier separating the ion-conducting liquid from the liquid-activated battery (1).

3. The device, according to claim 1, wherein the non ion-conducting PCM material (2) takes the form of microcapsules, which contain the ion-conducting liquid, and being the microcapsules in contact with the liquid-activated battery (1) and/or inserted in the liquid-activated battery (1).

4. The device, according to claim 1, wherein the non ion-conducting PCM material (2) takes the form of a porous substrate, wherein the ion-conducting liquid is inserted in the pores of the non ion-conducting PCM material (2), and being the non ion-conducting PCM material (2) placed in contact with the liquid-activated battery (1) and/or inserted in the liquid-activated battery (1).

5. A passive event detection device, that comprises:
- a support substrate (5),
- a liquid-activated battery (1), linked to the support substrate (5),
- an ion-conducting PCM material (2) (Phase Change Material), that is ion-conducting in its liquid phase and non ion-conducting in its solid phase, positioned in contact with the liquid-activated battery (1), changing the ion-conducting PCM material (2) from solid to liquid when an environmental physical or chemical magnitude exceeds a threshold in intensity and/or duration, activating the liquid-activated battery (1), and
- an electronics module (4), connected to the liquid-activated battery (1), that is powered up when the liquid-activated battery (1) is activated.

6. The device according to claim 5, wherein the ion-conducting PCM material (2) is placed outside the liquid-activated battery (1) and in direct contact with the liquid-activated battery (1).

7. The device according to claim 5, wherein the ion-conducting PCM material (2) is placed inside the liquid-activated battery (1) and/or in direct contact with the liquid-activated battery (1).

8. The device according to claims 1 or 5, wherein the electronics module (4) additionally comprises an emitter (6) that generates a signal when the electronics module (4) is powered up.

9. The device according to claim 8, wherein the emitter (6) produces a signal selected between a radiofrequency signal, a light, a sound and a vibration.

10. The device according to claims 1 or 5, wherein the electronics module (4) additionally comprises one or more sensors (8) that record a physical or chemical environmental magnitude when the electronics module (4) is powered up.

11. The device according to claims 1 or 5, wherein the electronics module (4) additionally comprises a memory that records information when the electronics module (4) is powered up.

12. The device according to claims 1 or 5, wherein the electronics module (4) additionally comprises one or more actuators that are activated when the electronics module (4) is powered up.

13. The device according to claims 1 or 5, wherein the liquid-activated battery (1) is a single use paper-battery, the substrate (5) is made out of a compostable material, and the electronics module (4) comprises a printed antenna (6) on the substrate (5), and a non-encapsulated chip connected to the antenna (6) and the liquid-activated battery (1).

14. The device according to claims 1 or 5, wherein the PCM material (2) is a material that changes from solid to liquid when a magnitude selected from physical parameters such as temperature, mechanical stress, hydrostatic pressure, electromagnetic field, electrical field, radiation, and radioactivity to chemical parameters such as gas composition, water absorption, and pH.

15. The device according to claims 1 or 5, wherein the electronics module (4) additionally comprises a geolocalizer that geolocalizes the device when the electronics module (4) is powered up.
